(19) Europäisches Patentamt
European Patent Office
Office européen des brevets

(11) Veröffentlichungsnummer: **0 023 545**
**B1**

(12) # EUROPÄISCHE PATENTSCHRIFT

(45) Veröffentlichungstag der Patentschrift: **21.04.82**

(51) Int. Cl.³: **C 07 C 179/133**, C 07 C 178/00

(21) Anmeldenummer: **80103162.6**

(22) Anmeldetag: **09.06.80**

(54) **Verfahren zur Herstellung aromatischer Peroxycarbonsäuren.**

(30) Priorität: **23.07.79 DE 2929839**

(43) Veröffentlichungstag der Anmeldung: **11.02.81 Patentblatt 81/6**

(45) Bekanntmachung des Hinweises auf die Patenterteilung: **21.04.82 Patentblatt 82/16**

(84) Benannte Vertragsstaaten: **AT BE CH DE FR GB IT LI NL SE**

(56) Entgegenhaltungen:
**US-A-4 119 660**
**JOURNAL OF ORGANIC CHEMISTRY, Band 27, 1962**
**L. S. SILBERT et al.: »Peroxides. IX. New method for the direct preparation of aromatic and aliphatic peroxy acids« Seiten 1336—1342**

(73) Patentinhaber: **Degussa Aktiengesellschaft, Weissfrauenstrasse 9, D-6000 Frankfurt am Main 1 (DE)**
Patentinhaber: **Henkel Kommanditgesellschaft auf Aktien, -Patentabteilung- Postfach 1100 Henkelstrasse 67, D-4000 Düsseldorf 1 (DE)**

(72) Erfinder: **Stober, Reinhard, Dr. Dipl.-Chem., Ludwig-Uhland-Strasse 7, D-6451 Grosskrotzenburg (DE)**
Erfinder: **Wirthwein, Rolf, Dr. Dipl.-Chem., Fürstenbergstrasse 4, D-6450 Hanau 9 (DE)**

**EP 0 023 545 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung aromatischer Peroxycarbonsäuren

Es ist seit langem bekannt, daß sich aliphatische Peroxycarbonsäuren durch Umsetzung der betreffenden aliphatischen Carbonsäuren mit Wasserstoffperoxid in Gegenwart von Schwefelsäure herstellen lassen, s. zum Beispiel Swern, Chemical Reviews, Bd. 45, 1949, Seite 5; Silbert und Mitarbeiter, J. Org., Chem., 27 (1962), Seite 1336.

Während sich dieses Verfahren als erfolgreich bei der Gewinnung aliphatischer Peroxycarbonsäuren erwies, führten wiederholte Versuche, es auf die Umsetzung von aromatischen Säuren mit Wasserstoffperoxid zu übertragen, nicht zum gewünschten Ergebnis. Entweder erfolgten Decarboxylierung oder Sulfonierung/Hydroxylierung des aromatischen Rings bzw. totale oxidative Zerstörung, s. loc. cit. Silbert, Seite 1337; US-PS 3 143 562; DE-AS 1 155 113; GB-PS 930 056; Methodicum Chimicum, Bd. 5 (1975) C-O-Verbindungen, Seite 739; US-PS 4 147 720.

Die Fachwelt stand also bis in die jüngste Zeit unter dem Eindruck, daß Schwefelsäure als Lösungsmittel oder auch nur als Katalysatorsäure für die Herstellung von aromatischen Peroxycarbonsäuren ungeeignet sei.

Um diesen Schwierigkeiten zu begegnen, wurde das System »aromatische Carbonsäure — Wasserstoffperoxid — Schwefelsäure« durch das System »aromatische Säure — Wasserstoffperoxid — Methansulfonsäure« ersetzt, d. h. anstelle von Schwefelsäure wurde Methansulfonsäure verwendet, s. Silbert loc. cit.

Methansulfonsäure ist aber im Gegensatz zu Schwefelsäure keine technisch leicht zugängliche Grundstoffchemikalie und stellt dadurch für die technische Durchführung des Verfahrens eine unbedingte Belastung dar.

Da aromatische Peroxycarbonsäuren auch zu den wichtigen Oxidationsmitteln zählen und z. B. bei der Epoxidation und auch bei der Oxidation von Sulfiden oder tert. Aminen, sowie als Bleich- und Desinfektionsmittel eine Rolle spielen, wäre es sehr erwünscht, ein Verfahren zu besitzen, das ohne technisch aufwendige Materialien aromatische Peroxycarbonsäuren herzustellen gestattet.

Zweck der Anmeldung ist daher ein Verfahren zur Herstellung von aromatischen Peroxycarbonsäuren unter Verwendung technisch leicht zugänglicher Mittel.

Es wurde nun gefunden, daß sich aromatische Peroxycarbonsäuren durch Umsetzen der betreffenden Carbonsäure mit Wasserstoffperoxid in Gegenwart von Schwefelsäure herstellen lassen, wenn man ein Gemisch von wäßrigem 50—99gew.-%igem $H_2O_2$ und Schwefelsäure im Reaktionsgemisch vorlegt und in dieses Gemisch die feste aromatische Carbonsäure unter Rühren einträgt, die Reaktion in heterogener Phase durchführt, das Rühren bis zur beendeten Reaktion fortsetzt und dann das erhaltene Reaktionsgemisch in bekannter Weise aufarbeitet.

Als aromatische Carbonsäure kommen sowohl ein- und mehrbasische, bevorzugt zweibasische Carbonsäuren, in Frage, wie Benzoesäure, Phthalsäuren, die Benzol-di- und poly-Carbonsäuren sowie ihre Derivate entsprechend der allgemeinen Formel

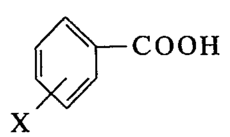

worin X = 1 bis 5 COOH-Gruppierungen, Alkyl (0—4 C), Alkoxy (1—4 C) und/oder Halogen bedeuten.

Die Carbonsäure bzw. ihre Derivate können auch als Mischungen eingesetzt werden.

Da die aromatischen Carbonsäuren bei Zimmertemperatur in fester Form vorliegen, findet das erfindungsgemäße Verfahren also in heterogener Phase statt. Besondere Korngrößen der aromatischen Carbonsäuren sind für das Verfahren nicht erforderlich. Die Umsetzung verläuft jedoch um so besser, je kleiner die Korngröße der eingesetzten aromatischen Carbonsäure ist.

Wasserstoffperoxid wird bevorzugt in Konzentrationen von 70 bis 85 Gew.-%, die Schwefelsäure in Konzentrationen von 50 bis 100 Gew.-%, bevorzugt von 96 Gew.-%, verwendet.

Das Molverhältnis von Wasserstoffperoxid zu Schwefelsäure schwankt in dem vorzulegenden Gemisch in der Regel von 0,67 bis 2 : 1. Die Zugabe von mehr Wasserstoffperoxid führt zu keiner nennenswerten Ausbeuteerhöhung.

Die aromatische Carbonsäure wird in fester Form, vorzugsweise pulverisiert, in die vorgelegte Mischung eingetragen.

Das Molverhältnis »Carbonsäure zu Wasserstoffperoxid zu Schwefelsäure« liegt in der Regel zwischen 1 : 2 : 3 bis 1 : 10 : 10. Innerhalb dieser Grenzen ist es beliebig einstellbar. Auch hier führen höhere Molverhältnisse nur zu unwesentlichen Ausbeutesteigerungen.

Die Umsetzung findet in der Regel bei Ausgangstemperaturen von 20 bis 70° C statt, bevorzugt bei 40—60° C.

Das Eintragen der Carbonsäure kann per Hand oder durch übliche Dosiervorrichtungen erfolgen.

Die Dauer des Eintragens hängt sowohl von der Art der einsetzenden Reaktion, d. h., ob stärker oder schwächer einsetzend, von der Korngröße der aromatischen Carbonsäure, d. h. je kleiner, desto

schneller, und von der freiwerdenden Reaktionswärme ab. Dabei spielt auch das jeweilige Molverhältnis von Carbonsäure zu Wasserstoffperoxid und Schwefelsäure eine Rolle.

Je geringer die Reaktionswärme ist bzw. je höher das oben definierte Molverhältnis, desto rascher ist das Eintragen beendet.

So kommt man z. B. bei der Gewinnung von Diperoxyisophthalsäure, die in einem Molverhältnis zu Wasserstoffperoxid und Schwefelsäure von 1 : 5 : 5 eingesetzt wird, für das Eintragen der festen Carbonsäuren mit wenigen Minuten aus. Die Reaktionswärme ist dabei vernachlässigbar gering, im Gegenteil, das Reaktionsgemisch wird zur vollständigen Umsetzung noch erwärmt.

Unter »Rühren« wird bevorzugt eine turbulente Durchmischung verstanden.

Die Aufarbeitung des Reaktionsgemisches erfolgte in bekannter Weise, z. B. nach US-PS 3 880 914; Parker et al. J. Am. Chem. Soc., 77 (1955), S. 4037 ff. und 79 (1957) 1929 ff.; Silbert et al., J. Org. Chem. 27 (1962) S. 1336 ff.; US-PS 4 147 720.

Das erfindungsgemäße Verfahren eignet sich besonders gut zur Herstellung der Benzoldiperoxycarbonsäuren (Diperoxyphthalsäuren).

Nach dem erfindungsgemäßen Verfahren werden die aromatischen Peroxycarbonsäuren im allgemeinen in Ausbeuten von 10 bis 80 Gew.-% mit Peroxysäuregehalten von 5 bis 90 Gew.-% erhalten.

Schon Ausbeuten von 10 Gew.-% bedeuten bei manchen aromatischen Peroxycarbonsäuren eine erhebliche Steigerung, da z. B. Diperoxyterephthalsäure selbst bei Verwendung von Methansulfonsäure nur in Ausbeuten unter 5 Gew.-% erhalten wurde. Dagegen wurde sie nach dem erfindungsgemäßen Verfahren mit einer Ausbeute von 14 Gew.-% gewonnen, s. DE-AS 1 155 113.

Zwar ist es nach dem Verfahren der DE-AS 1 048 569 bzw. US-PS 2 813 896 bekannt, Peroxycarbonsäuren und auch aromatische Peroxycarbonsäuren in Gegenwart von Schwefelsäure herzustellen. Es wird aber ausdrücklich darauf Wert gelegt, daß die Reaktion in homogener Lösung vorgenommen wird. Daher wird z. B. Peroxybenzoesäure in einer wäßrig-ätherischen Lösung hergestellt, ein Verfahren, das sicherheitstechnisch bedenklich und außerdem sehr zeitraubend ist.

Ebenso erwies sich ein stark wäßriges Gemisch aus 35gew.-%igem Wasserstoffperoxid und 95—98gew.-%iger Schwefelsäure — wie es nach US-PS 4 013 581 zur Herstellung von Diperoxyazelainsäure verwendet wird — zur Herstellung aromatischer Peroxycarbonsäuren als völlig ungeeignet. Es trat keine Umsetzung zu der gewünschten Peroxycarbonsäure ein.

Konzentriertere Lösungen, mit z. B. 50gew.-%igem Wasserstoffperoxid, führten nach Silbert, Siegel und Swern, J. Org. Chem., 27, (1962), Seite 1336 ff. zu Zersetzungen der eingesetzten, aromatischen Carbonsäuren.

So wurde auch bis in die jüngste Zeit immer von der Verwendung von Schwefelsäure zur Herstellung aromatischer Peroxycarbonsäuren abgeraten (siehe loc. cit. Methodicum Chimicum und US-PS 4 147 729). Statt dessen wurden spezielle Aufwendungen selbst bei Verwendung von Methansulfonsäure für nötig gehalten, wie z. B. spezielle Korngrößen der eingesetzten Phthalsäuren, s. US-PS 3 655 738.

Es war also sehr überraschend, daß entgegen der Ansicht der Fachleute eine Möglichkeit bestand, aromatische Peroxycarbonsäuren, bevorzugt aromatische Peroxydicarbonsäuren, in guten Ausbeuten und mit hohen Peroxysäuregehalten in technisch einfacher Weise mit dem System »aromatische Carbonsäure — Wasserstoffperoxid — Schwefelsäure« herzustellen.

Die Erfindung wird anhand folgender Beispiele beschrieben:

### Beispiel 1

### Diperoxyphthalsäure (DPP)

Zu einem Gemisch aus 51 g 96%iger $H_2SO_4$ (0,5 mol) und 28,4 g 60%igem $H_2O_2$ (0,5 mol) werden bei 20°C innerhalb von 10 min 17 g pulverisierte Phthalsäure (0,1 mol) unter Rühren zugegeben, die Reaktionsmischung auf 40°C erwärmt und bei dieser Temperatur 6 Stunden gerührt. Nach dem Abkühlen auf 10—15°C werden 150 ml gesättigte $(NH_4)_2SO_4$-Lösung zugegeben, die Reaktionsmischung filtriert und der Rückstand mit Eiswasser nachgewaschen. Die Trocknung des Filterrückstandes liefert 15,5 g Produkt mit einem Gehalt an Diperoxyphthalsäure von 49,3% (jodometrisch), entsprechend einer Ausbeute von 38,4%.

## Beispiel 2

## Diperoxyisophthalsäure (DPIP)

### Allgem. Vorschrift

Zu einem Gemisch von $H_2O_2$ und $H_2SO_4$ wird unter Rühren pulverisierte Isophthalsäure innerhalb 5—20 min gegeben und bei der jeweiligen Reaktionstemperatur 2—40 Stunden gerührt. Nach dem Abkühlen auf 0—10° C werden 200 ml Eiswasser zugegeben, die Reaktionsmischung filtriert und der Rückstand mit Eiswasser bis zu einem pH-Wert von 3—4 gewaschen. Nach der Trocknung des Produkts erhält man ein Produkt, das außer Diperoxyisophthalsäure nur noch Ausgangsmaterial enthält (Isophthalsäure).

Die Versuchsdurchführungen, die zu den Ergebnissen in den Tabellen 1—4 führten, wurden beispielhaft im Hinblick auf die Mengenansätze und Reaktionsbedingungen für die Versuche 3 und 4 der Tabelle 1 im folgenden angegeben:

Zu Versuch 3:

| | |
|---|---|
| Ansatz | 120 g 85%iges $H_2O_2$ (3,0 mol) |
| | 306 g 96%ige $H_2SO_4$ (3,0 mol) |
| | 99,7 g Isophthalsäure (0,6 mol) |
| Reaktionstemperatur | 50° C |
| Reaktionszeit | 23 Stunden |
| Ausbeute | 104 g mit einem Gehalt von DPIP von 88,7% entsprechend einer Ausbeute von 77,5%. |

Zu Versuch 4:

| | |
|---|---|
| Ansatz | 80 g 85%iges $H_2O_2$ (2,0 mol) |
| | 204 g 96%ige $H_2SO_4$ (2,0 mol) |
| | 66,4 g Isophthalsäure (0,4 ml) |
| Reaktionstemperatur | 60° C |
| Reaktionszeit | 7,5 Stunden |
| Ausbeute | 64,2 g mit einem Gehalt von DPIP von 72,3% entsprechend einer Ausbeute von 58,6%. |
| Elementaranalyse | |
| gefunden | 51,02%, H 3,32%; |
| berechnet (mit 72,3% DPIP) | 51,09%, H 3,21%. |
| Schwefelnachweis | negativ |
| Dünnschichtchromatogramm | 2 Komponenten |

(Laufmittel: tert.-Butanol/Eisessig/$H_2O$ = 4 : 1 : 1)

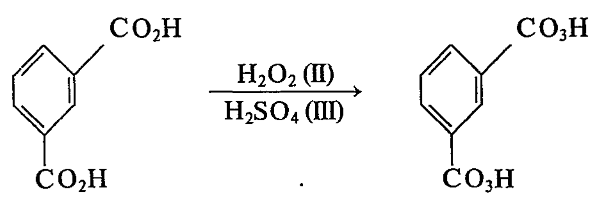


(I) (IV)

Tabelle 1

Verwendung von 85gew.-%igem $H_2O_2$

Molverhältnisse I : II : III = 1 : 5 : 5

| | Reaktionstemp. °C | Reaktionszeit h | DPIP-Geh. % | DPIP-Ausb. % |
|---|---|---|---|---|
| 1 | 60[a]) | 2,2 | 60,8 | 46,7 |
| 2 | 60 | 7,5 | 72,3 | 59,0 |
| 3 | 50[b]) | 7 | 80,0 | 67,0 |
| 4 | 50 | 23 | 88,7 | 77,5 |
| 5 | 40 | 23 | 83,2 | 73,5 |
| 6 | 40[b]) | 39 | 85,5 | nicht best.[c]) |
| 7 | 30 | 23 | 43,5 | 38,5 |

[a]) kurzzeitig 71 °C.
[b]) zusätzlich 16 h bei Raumtemperatur.
[c]) Ausbeute nicht bestimmbar, da Ansatz mehrfach geteilt.

Tabelle 2

Verwendung von 85,0gew.-%igem $H_2O_2$

verschiedene Molverhältnisse

| Molverh. I : II : III | Reakt.-Temp. °C | Reakt.-Zeit h | DPIP-Geh. % | DPIP-Ausb. % |
|---|---|---|---|---|
| 1 : 10 : 5 | 40 | 6 | 18,5 | 15,3 |
| 1 : 4 : 4 | 50 | 23 | 79,9 | 71,0 |
| 1 : 4 : 4 | 40 | 23 | 73,8 | 67,3 |
| 1 : 3 : 5 | 40 | 23 | 35,3 | 26,8 |
| 1 : 2,5 : 3 | 40 | 23 | 52,0 | 45,0 |
| 1 : 2 : 3 | 40 | 23 | 33,5 | 27,5 |

Tabelle 3

Verwendung von 70gew.-%igem $H_2O_2$

Molverhältnis I : II : III = 1 : 5 : 5

| Reaktionstemp. °C | Reaktionszeit h | DPIP-Gehalt % | DPIP-Ausb. % |
|---|---|---|---|
| 55 | 23 | 59,9 | 52,4 |
| 50 | 23 | 64,5 | 57,8 |
| 50[a]) | 23 | 48,0 | 42,0 |
| 40 | 23 | 43,7 | 38,6 |

[a]) Zusatz von 1-Hydroxyethan-1,1-diphosphonsäure (60gew.-%ige, wäßrige Lösung).

Tabelle 4

Verwendung von 70,0gew.-%igem $H_2O_2$

verschiedene Molverhältnisse

| Molverh. I : II : III | Reaktionstemp. °C | Reaktionszeit h | DPIP-Gehalt % | DPIP-Ausbeute % |
|---|---|---|---|---|
| 1 : 10 : 5 | 40 | 6 | 1,5 | 1,4 |
| 1 : 4 : 4 | 40 | 23 | 35,6 | 30,8 |
| 1 : 3 : 5 | 40 | 23 | 45,3 | 38,5 |
| 1 : 2,5 : 3 | 50 | 23 | 40,3 | 34,8 |
| 1 : 2 : 3 | 50 | 23 | 28,8 | 23,9 |

Beispiel 3

Diperoxyterephthalsäure (DPTP)

Allgem. Vorschrift wie unter Beispiel 2

Typisches Beispiel:

| | |
|---|---|
| Ansatz | 20 g 85%iges $H_2O_2$ (0,5 mol)<br>51 g 96%iges $H_2SO_4$ (0,5 ml)<br>17 g Terephthalsäure (0,1 mol) |
| Reaktionstemperatur | 50° C |
| Reaktionszeit | 23 Stunden |
| Ausbeute | 17 g mit einem Gehalt an DPTP von 15,8% entsprechend einer Ausbeute von 13,6%. |

**Patentansprüche**

1. Verfahren zur Herstellung aromatischer Peroxycarbonsäuren durch Umsetzung von aromatischen Carbonsäuren mit Wasserstoffperoxid in Gegenwart von Schwefelsäure, dadurch gekennzeichnet, daß man ein Gemisch von wäßrigem 50—99gew.-%igem $H_2O_2$ und Schwefelsäure in dem

Reaktionsgefäß vorlegt, darauf unter Rühren die feste aromatische Carbonsäure einträgt, die Reaktion in heterogener Phase durchführt, das Rühren bis zur beendeten Reaktion fortsetzt und dann das erhaltene Reaktionsgemisch in bekannter Weise aufarbeitet.

2. Verfahren nach Anspruch 1, dadurch gekennzeichnet, daß man Wasserstoffperoxid und Schwefelsäure im Molverhältnis von 0,67 bis 2 : 1 vorlegt.

3. Verfahren nach Anspruch 1—2, dadurch gekennzeichnet, daß man ein Molverhältnis von aromatischer Carbonsäure zu Wasserstoffperoxid zu Schwefelsäure von 1 : 2 : 3 bis 1 : 10 : 10 einsetzt.

4. Verfahren nach Anspruch 1—3, dadurch gekennzeichnet, daß man die Reaktionen bei Ausgangstemperaturen von 40—60° C durchführt.

5. Verfahren nach Anspruch 1—4, dadurch gekennzeichnet, daß man als aromatische Carbonsäure eine der Phthalsäuren einsetzt.

6. Verfahren nach Anspruch 1—5, dadurch gekennzeichnet, daß man als aromatische Carbonsäure Isophthalsäure einsetzt.

7. Verfahren nach Anspruch 1—5, dadurch gekennzeichnet, daß man als aromatische Carbonsäure Phthalsäure einsetzt.

## Claims

1. A process for the production of aromatic peroxy carboxylic acids by reacting aromatic carboxylic acids with hydrogen peroxide in the presence of sulfuric acid, characterised in that a mixture of 50 to 99% by weight $H_2O_2$ and sulfuric acid is initially introduced into the reaction vessel, after which the solid aromatic carboxylic acid is introduced with stirring, the reaction is carried out in heterogeneous phase, stirring is continued until the reaction is over and the reaction mixture obtained is subsequently worked up in known manner.

2. A process as claimed in Claim 1, characterised in that hydrogen peroxide and sulfuric acid are initially introduced in a molar ratio of from 0.67 to 2 : 1.

3. A process as claimed in Claims 1 and 2, characterised in that the aromatic carboxylic acid, the hydrogen peroxide and the sulfuric acid are used in a molar ratio of from 1 : 2 : 3 to 1 : 10 : 10.

4. A process as claimed in Claims 1 to 3, characterised in that the reactions are carried out at starting temperatures of from 40 to 60° C.

5. A process as claimed in Claims 1 to 4, characterised in that one of the phthalic acids is used as the aromatic carboxylic acid.

6. A process as claimed in Claims 1 to 5, characterised in that isophthalic acid is used as the aromatic carboxylic acid.

7. A process as claimed in Claims 1 to 5, characterised in that phthalic acid is used as the aromatic carboxylic acid.

## Revendications

1. Procédé pour la fabrication d'acides peroxycarboxyliques aromatiques par réaction d'acides carboxyliques aromatiques avec du peroxyde d'hydrogène en présence d'acide sulfurique, caractérisé en ce qu'on prépare, dans le réacteur, un mélange d'eau oxygénée à 50 à 99 pour cent en poids en sulution aqueuse et d'acide sulfurique, puis on introduit, tout en agitant, l'acide carboxylique aromatique solide, on effectue la réaction en phase hétérogène, on poursuit l'agitation jusqu'à ce que la réaction soit terminée, et on traite ensuite le mélange réactionnel obtenu de la manière habituelle.

2. Procédé suivant la revendication 1, caractérisé en ce que l'on met en oeuvre le peroxyde d'hydrogène, et l'acide sulfurique dans le rapport moléculaire de 0,67 à 2/1.

3. Procédé suivant l'une des revendications 1 et 2, caractérisé en ce que l'on met en oeuvre un rapport moléculaire de l'acide carboxylique aromatique par rapport au peroxyde d'hydrogène et à l'acide sulfurique de 1/2/3 à 1/10/10.

4. Procédé suivant l'une des revendications 1 à 3, caractérisé èn ce que l'on effectue les réactions à des températures de départ de 40 à 60° C.

5. Procédé suivant l'une quelconque des revendications 1 à 4, caractérisé en ce que l'on utilise comme acide carboxylique l'un des acides phthaliques.

6. Procédé suivant l'une quelconque des revendications 1 à 5, caractérisé en ce qu'on utilise comme acide carboxylique aromatique, l'acide isophthalique.

7. Procédé suivant l'une quelconque des revendications 1 à 5, caractérisé en ce que l'on utilise comme acide carboxylique aromatique, l'acide phthalique.